# EUROPÄISCHE PATENTANMELDUNG

(11) **EP 0 905 252 A2**
(43) Veröffentlichungstag der Anmeldung: **31.03.1999**
(21) Anmeldenummer: 98114218.5
(22) Anmeldetag: 07.07.1993
(51) Int. Cl.: C12N 15/82, A01H 5/00

(54) **Modulartiges Promotor-Konstrukt**

(30) Priorität: 08.07.1992 DE 4222407
(62) Teilanmeldung aus: 93915833.3
(71) Anmelder: Max-Planck-Gesellschaft zur Förderung der Wissenschaften e.V., D-37073 Göttingen (DE)
(72) Erfinder: Maas, Christoph, Dr., 60933 Frankfurt (DE); Schell, Jeff, Prof., Dr., 50829 Köln (DE); Steinbiss, Hans-Henning, Dr., 53804 Much (DE)
(74) Vertreter: Oser, Andreas

(57) **Zusammenfassung**

Es wird ein modulartiges Promotor-Konstrukt beschrieben, das einen in Pflanzenzellen wirksamen Promotor und eine DNA-Sequenz aus dem Exon 1 des Aktin 1-Gens aus Reis aufweist. Dieses modulartige Promotor-Konstrukt führt ggf. mit weiteren regulatorischen DNA-Sequenzen zu einer beträchtlichen Steigerung der Genexpression in Pflanzenzellen.

## Beschreibung

Die Erfindung betrifft ein modulartiges Promotor-Konstrukt auf der Basis des Exon 1 des Aktin 1-Gens aus Reis.

Es bestand schon immer das Bedürfnis, agronomisch wichtige Getreidepflanzen mit verbesserten Eigenschaften auszustatten. Früher hat man auf klassischem Wege Erbanlagen bzw. Gene in die betreffenden Pflanzen eingekreuzt. Seit Etablierung der Gentechnologie ist es möglich, definierte Gene in das Genom von Getreidepflanzen, wie beispielsweise Reis, Mais, Weizen und Gerste, einzuschleusen. Neben der Auswahl des richtigen selektiven Marker-Gens und der agronomisch wichtigen Gene ist die Wahl des richtigen Promotors zur effektiven Expression des gewünschten Gens maßgeblich. Der am meisten verwendete Promotor zur Erhöhung der Expression von chimären Genen in monokotyledonen Getreidepflanzen stammt aus dem Cauliflower Mosaic Virus (CaMV) 355 RNA-Gen (Odell et al., Nature 313, Seiten 810 bis 820, 1985). Dieser Promotor ist jedoch im Vergleich zu dikotyledonen Pflanzen relativ wenig aktiv in monokotyledonen Pflanzen (Töpfer et al., Meth. Enzymol., "Rec. DNA", im Druck). So sind alternative Genexpressionsvektoren unter Verwendung starker Promotoren, wie die Promotoren des Aktin 1-Gens aus Reis (Mc Elroy et al., Plant Cell 2, Seiten 163 bis 171, 1990) und des Ubiquitin-Gens aus Mais (Christensen and Fox, International Society for Plant Molecular Biology Meeting, Program Abstracts, No. 287, 1991) beschrieben worden.

In Analogie zu tierischen Systemen (Dynan, Cell 58, Seiten 1 bis 4, 1989) ist es ebenfalls für Pflanzengene wahrscheinlich, daß die durch die RNA-Polymerase II vermittelte Transkription von modulartigen, d.h. aus verschiedenen regulierenden DNA-Sequenzen bestehenden Elementen abhängt. Es ist bereits für eine Anzahl von verschiedenen modulartigen Elementen gezeigt worden, daß sie eine wichtige Rolle bei z.B. der gewebespezifischen Transkription spielen (Katagiri und Chua, Trends Gent. 8, Seiten 22 bis 70, 1992).

So ist aus der DE-OS 41 24 537 eine modulartige Promotor-Konstruktion bekannt, mit der die Genexpression fremder Gene in Pflanzenzeilen gesteigert werden kann. Dabei wird eine DNA-Sequenz aus dem Exon 1 des Saccharosesynthase-Gens aus *Zea mays L*. zwischen Promotor und das zu exprimierende Gen eingesetzt. Eine weitere multiplikative Steigerung der Genexpression ist möglich, wenn an die genannte DNA-Sequenz eine weitere DNA-Sequenz gekoppelt wird, die im wesentlichen dem Intron 1 aus dem Saccharosesynthase-Gen aus *Zea mays L*. entspricht.

Die Isolierung von in der 5'-Region liegenden Promotorsequenzen des Aktin 1-Gens aus Reis ist von Mc Elroy et al., a.a.O., beschrieben worden. Es hat sich herausgestellt, daß eine positive Korrelation zwischen den Promotorsequenzen und den nachfolgenden Intronsequenzen besteht. So hat sich ein Promotor-Konstrukt aus diesen Sequenzen bei Transformationsuntersuchungen von Reis-Protoplasten als wirksamer Regulator der konstitutiven Expression eines fremden Gens erwiesen. Um das Muster und den Grad der Aktivität der 5'-Region des Aktin 1-Gens zu bestimmen, wurden von W. Zhang et al. (Plant Cell 3, Seiten 1155 bis 1165, 1991) transgene Reispflanzen erzeugt, die die 5'-Region des Aktin 1-Gens enthielten, welche an eine kodierende Sequenz der bakteriellen β-Glucuronidase als Fremdgen geknüpft war.

Der vorliegenden Erfindung liegt die Aufgabe zugrunde, eine Pflanze mit einem Promotor auszustatten, mit dem man fremde Gene in der Pflanze mit hoher Effizienz exprimieren kann.

Diese Aufgabe wird durch eine Pflanze gemäß Patentanspruch 1 gelöst. Gegenstand der Erfindung ist auch das Vermehrungsgut der Pflanze.

Die Unteransprüche betreffen bevorzugte Ausführungsformen des Promotor-Konstrukts, mit dem die erfindungsgemäße Pflanze transformiert ist.

Das in der Erfindung eingesetzte, modulartige Promotor-Konstrukt weist einen in Pflanzenzellen wirksamen Promotor und eine DNA-Sequenz aus dem Exon 1 des Aktin 1-Gens aus Reis auf und schließt die Allele sowie die Genexpression stimulierende Derivate dieses modulartigen Promotor-Konstrukts ein.

Die Figuren dienen zur Erläuterung der Erfindung. Es zeigen:
- **Fig. 1**: chimäre Genkonstrukte für transiente Genexpressionsexperimente,
- **Fig. 2**: eine schematische Darstellung des Aktin 1 (Akt 1)-Gens aus Reis mit der verwendeten Exon 1-DNA-Sequenz,
- **Fig. 3**: ein Dünnschichtchromatogramm der transienten Expression von chimären Konstrukten in *Hordeum vulgare* Protoplasten,
- **Fig. 4**: ein Dünnschichtchromatogramm der transienten Expression von chimären Konstrukten in *Triticum monococcum* Protoplasten und
- **Fig. 5**: ein Dünnschichtchromatogramm einer weiteren transienten Genexpression in *Hordeum vulgare* Protoplasten.

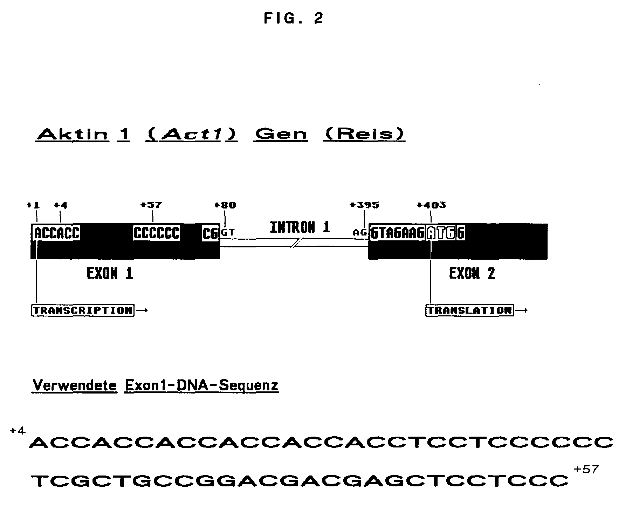

Bei dem erfindungsgemäß eingesetzten Promotor-Konstrukt handelt es sich um einen die RNA-Polymerase II stimulierenden Promotor. Das modulartige Promotor-Konstrukt weist einen in Pflanzenzellen wirksamen Promotor und eine daran gekoppelte regulatorische DNA-Sequenz auf. Diese DNA-Sequenz stammt aus dem Exon 1 des Aktin 1-Gens (Akt1-Exon1-Sequenz) aus Reis. Die im Promotor-Konstrukt enthaltene DNA-Sequenz entspricht vorzugsweise der Sequenz von der Position +4 bis +57 im Exon 1 des Aktin 1-Gens, wie aus Fig. 2 zu entnehmen ist. Das untranslatierte Exon 1 ist vom Startpunkt der Translation in Exon 2 durch ein Intron (Intron 1) getrennt. Das Exon 1 liegt somit in der 5'-transkribierten, jedoch untranslatierten Region des Aktin 1-Gens. Die gesamte Sequenz des Exon 1 des genannten Gens ist bei Mc Elroy et al., (a.a.O.) beschrieben.

Die Akt1-Exon1-Sequenz ist GC-reich (77 %) und wirkt als die RNA-Polymerase II stimulierendes Element, wenn es sich in downstream-Richtung der Transkriptionsstartstelle befindet.

Es wurde gefunden, daß, wenn das modulartige Promotor-Konstrukt an ein in einer Pflanzenzelle zu exprimierendes Gen gekoppelt ist, die Expression dieses Gens erheblich gesteigert wird, wobei eine mindestens 10-fache Steigerung der Genexpression festgestellt werden konnte.

Die Genexpression kann weiterhin um das 100-fache erhöht werden, wenn die Akt1-Exon1-Sequenz mit dem Intron 1 des Saccharosesynthase-Gens aus *Zea mays* kombiniert wird, was überraschenderweise zu einer Erhöhung der Genexpression auf bis mindestens das 1000-fache führt.

Es hat sich herausgestellt, daß die 18 bp OTF-Bindungsstelle des Promotors der Octopinsynthase (OCS-Enhancer) beispielsweise mit dem CaMV 35S-Promotor kompatibel ist. Wenn man die OTF-Bindungsstelle mit der Akt1-Exon1-Sequenz und dem Intron 1 aus dem Saccharosesynthase-Gen aus *Zea mays L*. kombiniert, erhält man eine sprunghafte Steigerung der Genaktivität um mindestens das 4000-fache.

Aus dem Vorstehenden ergibt sich, daß sich die Genexpression in Abhängigkeit von der mit dem zu exprimierenden Gen gekoppelten regulativen DNA-Sequenz multipliziert. Somit kann mit dem erfindungsgemäßen modulartigen Promotor-Konstrukt eine Multiplikation der Genaktivität erreicht werden.

Die erfindungsgemäß eingesetzten modulartigen Promotor-Konstrukte sowie die Vektoren enthalten als einen Pflanzenzellen wirksamen Promotor, wie beispielsweise den CaMV 35S-Promotor, den Nopalinsynthase-Promotor oder den Saccharosesynthase-Promotor.

Das modulartige Promotor-Konstrukt eignet sich für die Expression aller Arten von Fremdgenen, wie beispielsweise Resistenzgenen, wie sie z.B. aus der EP-A 0 257 542 und der EP-A 0 275 957 bekannt sind, wie auch zur Herstellung von proteinogenen Wirkstoffen in Pflanzen beispielsweise nach DD-A 12 65 164. Von besonderer Bedeutung sind Gene für Speicherproteine, wie z.B. Zeine oder Hordeine.

In downstream-Richtung an das zu exprimierende Gen enthalten die modulartigen Promotor-Konstrukte sowie die Vektoren, die die erfindungsgemäßen Promotor-Konstrukte enthalten, Polyadenylierungsregionen aus dem CaMV 35S- oder Octopinsynthase (OCS)-Gen (Hein et al., Mol. Gen. Genet, 199, Seiten 161 bis 168 (1985).

Das modulartige Promotor-Konstrukt eignet sich zur Expression fremder Gene in monokotyledonen und dikotyledonen Pflanzen. Als monokotyledone Pflanzen kommen beispielsweise Getreidepflanzen, wie Weizen, Gerste, Mais und Reis in Frage. Als dikotyledone Pflanze läßt sich z.B. Tabak (*Nicotiana tabaccum*) in zufriedenstellender Weise transformieren.

Es ist selbstverständlich, daß im Rahmen der Erfindung auch allelische Varianten und Derivate des oben genannten modulartigen Promotor-Konstrukts erfaßt sind, unter der Voraussetzung, daß diese modifizierten modulartigen Promotor-Konstrukte die gleiche Funktion wie die oben genannten Promotor-Konstrukte ausüben, d.h. stimulierend auf die Genexpression wirken. Zu den allelischen Variationen und Derivaten zählen beispielsweise Deletionen, Substitutionen, Insertionen, Inversionen oder Additionen der einzelnen Sequenzen des Promotor-Konstrukts.

Die Transformation von Pflanzen mit einem fremden Gen unter Verwendung des modulartigen Promotor-Konstrukts kann mit Hilfe üblicher Transformationsverfahren durchgeführt werden. Besonders bevorzugt ist die Protoplastentransformation mit anschließender Regeneration der aus den transformierten Protoplasten entstandenen Pflanzenzellen zu einer Pflanze.

In den nachfolgenden Beispielen werden besonders bevorzugte Ausführungsformen der Erfindung beschrieben.

### Beispiel 1

### Plasmidkonstruktionen

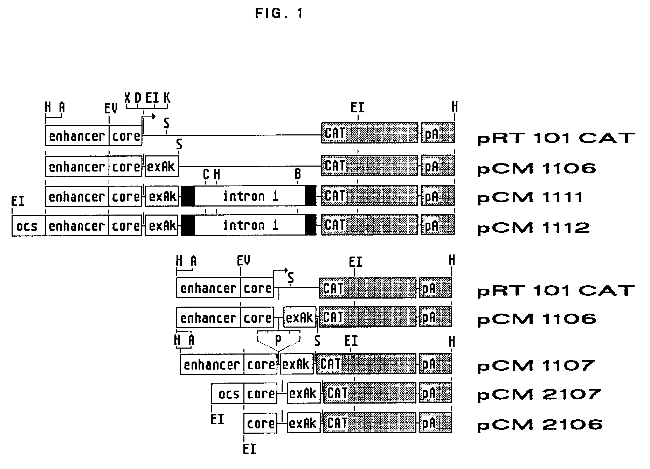

Die nachfolgend beschriebenen Plasmidkonstruktionen lassen sich der Fig. 1 entnehmen. Die dort gezeigten chimären Genkonstruktionen wurden für transiente Genexpressionsexperimente verwendet. Die Startstelle der Transkription und das Polyadenylierungssignal des CaMV 35S-Gens sind in allen Konstruktionen gleich. Die für das Klonieren relevanten Restriktionsstellen sind zusätzlich eingeführt worden. Der Polylinker (P) zwischen dem Transkriptions- und Translationsstart umfaßt vom 5'- zum 3'-Ende die Restriktionsstellen X, D, EI und K. Die Restriktionsstellen werden folgendermaßen abgekürzt: *Bcl*I (B), *Eco*RI (EI), *Eco*RV, (EV), *Hind*III (H), *Hinc*II (A), *Kpn*I (K), *Sma*I (S), *Sst*II (C), *Xho*I (X). Der CaMV 35S-Promotor ist mit "enhancer core" und die OTF-Bindungsstelle mit "OCS" gekennzeichnet.

Sämtliche Plasmidkonstruktionen wurden nach üblichen Verfahren, wie sie beispielsweise bei Sambrook et al, Molecular cloning: A laboratory manual, 2nd edition, Cold Spring Harbor Laboratory Press, Cold Spring Harbor, New York, 1989, beschrieben sind, hergestellt.

Alle Plasmidkonstruktionen leiten sich vom chimären Plasmid pRT 101 CAT ab (Pröls et al., Plant Cell, Rep. 7, Seiten 221 bis 224, 1988), welches das CAT-Markergen (CAT = Chloramphenicol-Transacetylase) enthält. Zur Herstellung des chimären Plasmids pCM 1106 wurde die nicht-translatierte Sequenz des Exon 1 des Aktin 1-Gens (Akt1-Exon1-Sequenz) von der Position +4 bis +57 in die einzige *Sma*I-Restriktionsstelle (S) im 5'-nicht translatierten Leader des pRT 101 CAT-Plasmids eingefügt. Hierzu sei auf die Fig. 2 verwiesen, woraus die verwendete Sequenz aus dem Exon 1 und ihre Position innerhalb des Exon 1 zu entnehmen ist. Bei Einführung der genannten DNA-Sequenz stellt man die *Sma*I-Restriktionsstelle durch 3'-terminale Cystosinreste des Aktin 1-Elements wieder her (siehe auch Fig. 2, Position +55 bis +57).

Man isoliert die Intron 1-Sequenzen aus dem Saccharosesynthase-Gen (*Sh1*) aus Mais (Position +43 bis +1084) aus dem aus der DE-OS 41 24 537 bekannten chimären Plasmid pSP 1076 + 1084 als *Hinc*II-Restriktionsfragment und fügt es in die *Sma*I-Restriktionsstelle von pCM 1106. Auf diese Weise erhält man die chimäre Plasmidkonstruktion pCM 1111.

Man synthetisiert mit einem DNA-Synthesizer (Applied Biosystem 380 B) die in Fig. 2 gezeigte Sequenz aus dem Exon 1 des Aktin 1-Gens aus Reis und die 18 bp OTF-Bindungsstelle mit der Sequenz AACGTAAGCGCTTACGTT (Ellis et al., EMBO J. 6, Seiten 3203 bis 3208, 1987). Man subkloniert dann die OTF-Bindungsstelle in die einzige *Sma*I-Restriktionsstelle des handelsüblichen Plasmids pUC 19 und erhält pOTF 18. Für die Herstellung der chimären Plasmide pCM 1112 und pCM 2107 isoliert man aus pCM 1111 und pCM 2106 ein *Hinc*II (A)/*Hind*III (H)-Restriktionsfragment (*Hind*III ist ein partieller Verdau von pCM 1111) und fügt es in die einzige *Hinc*II-Restriktionsstelle von pOTF 18 ein.

Für die Herstellung des Plasmids pCM 1107 entfernt man den Polylinker des chimären Plasmids pCM 1106 durch eine Restriktionsverdauung mit *Xho*I (X) / *Kpn*I (K). Man entfernt die überstehenden Enden mit einer vorsichtigen S1-Behandlung. Die Autolegierung führt zu dem Plasmidkonstrukt pCM 1107, welches im Vergleich zu pCM 1106 eine Deletion von 11 Basenpaaren zwischen der Stelle des Transkriptionsstartes und der Akt1-Exon1-Sequenz aufweist.

Das Promotordeletionsplasmid pCM 2106 wird hergestellt, indem CaMV 35S-Promotorsequenzen von pCM 1106 in upstream-Richtung der *Eco*RV (EV)-Restriktionsstelle bei Position -90 entfernt werden.

### Beispiel 2

### 2.1 Analyse der transienten Expression

Man führt nach der für Mais beschriebenen Methode (Maas und Werr, Plant Cell. Rep. 8, Seiten 148 bis 151, 1989) eine Protoplastenisolation aus einer Zellsuspension der Gerstenlinie *Hordeum vulgare* L. cv. Golden Promise mit der Maßgabe durch, daß man eine Osmolarität von 720 mosm verwendet. Die Aufzucht einer Zellsuspension der Linie *Triticum monococcum* (Einkorn) und Protoplastenisolation wurde wie im wesentlichen durch Lörz et al., Mol. Gen. Genet. 199, Seiten 178 bis 182, 1985 und Matzeit et al., Plant Cell 3, Seiten 247 bis 258, 1991) beschrieben, durchgeführt.

Die Transformation der *Hordeum vulgare* und *Triticum monococcum* Protoplasten wurde wie bei Maas und Werr a.a.O. und Maas et al., a.a.O., beschrieben durchgeführt. Man transformiert etwa 1 x 10⁶ Protoplasten mit 25 µg Plasmid-DNA und 100 µg sonifizierter Kalbsthymus-DNA und fügt einen durch PEG vermittelten Gentransfer durch (PEG-Lösung: 25 % PEG (1500), 0,1 M MgCl₂, pH 6,0). Man untersucht die Genexpression anhand des Gehalts an gebildetem Protein 15 bis 19 Stunden nach der Transformation.

### 2.2 Bestimmung der CAT-Aktivität

Man zentrifugiert die transformierten Zellen für die CAT-Aktivitätsbestimmung, resuspendiert in 60 µl 500 mM Tris/HCl bei einem pH-Wert von 7,5 (2 mM PMSF) und lysiert in zwei Zyklen unter Frieren und Tauen. Man klärt den Extrakt durch Zentrifugieren auf. Man verwendet 20 µl des Überstandes zur Proteinbestimmung gemäß Bradford, Anal. Biochem. 72, Seiten 248 bis 254, 1976. Die CAT-Aktivitätsbestimmung wird, wie im wesentlichen durch Gorman et al., Mol. Cell. Biol. 2, Seiten 1044 bis 1051, 1982 beschrieben, über Dünnschichtchromatographie durchgeführt. Die Extrakte wurden während 10 Minuten bei 65°C vorinkubiert, wobei 250 µg BSA in die Reaktionsmischung hinzugefügt wurde.

Für jedes Plasmidkonstrukt wurde parallel zwei Transformationen durchgeführt. Man kultiviert die behandelten Protoplasten und vereinigt sie, um die Unterschiede durch die verschiedenen Transformationsleistungen herabzusetzen. Man analysiert eine Hälfte (1 x 10⁶ Protoplasten) dieser Mischung auf CAT-Aktivität. Aufgrund der hohen CAT-Aktivitäten in unverdünnten Extrakten stellt man serienmäßig 1:10-Verdünnungen (1:10, 1:100, 1:1000) her, um somit lineare CAT-Aktivitäten für die densitometrische Untersuchung der Autoradiographien zu erhalten.

### 2.3 Untersuchungsergebnisse

### 2.3.1 Hordeum vulgare

Die Untersuchungen der Genexpression in *Hordeum vulgare* Protoplasten mit den in Beispiel 1 beschriebenen chimären Plasmiden hat zu folgenden Ergebnissen geführt.

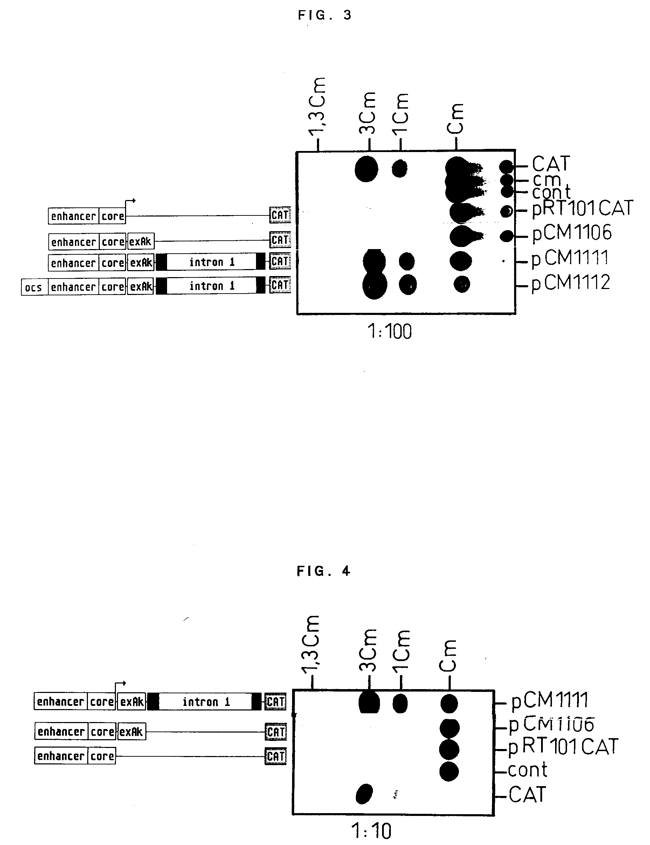

Die Ergebnisse der relativen CAT-Aktivitäten lassen sich dem Dünnschichtchromatogramm der Fig. 3 entnehmen. Aufgrund der hohen CAT-Aktivitäten im Rohextrakt wurde die Aktivität einer 1:100-Verdünnung mit 1 x 10⁴ Protoplasten gemessen. Die Abkürzungen haben folgende Bedeutungen: CAT = Chloramphenicol-Transacetylase; Cm = C¹⁴-markiertes Chloramphenicol allein; Cont = Proteinextrakte von nicht transformierten Kontrollprotoplasten und 1, 3, 1,3 Cm = an den angegebenen Positionen acetyliertes Chloramphenicol.

Es ergibt sich beim chimären Plasmid pCM 1106 im Vergleich zum Referenzplasmid pRT 101 CAT eine bis zu mindestens 10-fache Erhöhung der Markergenexpression.

Am chimären Plasmid pCM 1111 ist zu erkennen, daß, wenn die Akt1-Exon1-Sequenz mit den Intron 1-Sequenzen aus dem Saccharosesynthase-Gen kombiniert wird, die Markergenexpression auf das mindestens 1000-fache erhöht wird.

Die 18 bp OTF-Bindungsstelle des Octopinsynthase-Promotors mit dem gesamten CaMV 35S-Promotor kann mit der Akt1-Exon1-Sequenz sowie dem Intron 1 aus dem Saccharosesynthase-Gen interagieren. Die Einführung der OTF-Bindungsstelle in das chimäre Plasmid pCM 1111, welches die Akt1-Exon1-Sequenz und Sequenzen aus dem Intron 1 des Saccharosesynthase-Gen enthält, führt zu einer weiteren mindestens 3- bis 4-fachen Erhöhung des Stimulationsverhältnisses (siehe pCM 1112). Dieses Ergebnis erhält man auch mit einer 1:1000-fachen Verdünnung (nicht gezeigt). Somit ist eindeutig belegt, daß die stimulierende Wirkung der Kombination aus verschiedenen individuellen DNA-Sequenzen multiplikativ und nicht additiv ist.

In diesem Zusammenhang wird auf die nachfolgende Tabelle 1 verwiesen, worin die CAT-Aktivitäten aus Fig. 3 der chimären Plasmide pCM 1106, pCM 1111 und pCM 1112 in Relation zum Referenzplasmid pRT 101 CAT (Aktivität = 1) in *Hordeum vulgare* Protoplasten aufgeführt sind.

**Tabelle 1**

| pRT 101 CAT | pCM 1106 | pCM 1111 | pCM 1112 |
|---|---|---|---|
| 1 | 7,5-12,5 | 872-1242 | 3897-6985 |

### 2.3.2 Triticum monococcum

Die Untersuchungen der Genexpression in *Triticum monococcum* Protoplasten mit den in Beispiel 1 beschriebenen chimären Plasmiden hat zu folgenden Ergebnissen geführt.

Wie sich aus Fig. 4 entnehmen läßt, erreicht man mit transformierten *Triticum monococcum* Protoplasten gleiche Stimulationsverhältnisse der Genexpression wie mit *Hordeum vulgare* Protoplasten. So führt die Einfügung der Akt1-Exon1-Sequenz zu einer mindestens 10-fachen Erhöhung der Markergenexpression (pCM 1106). Die Kombination der Akt1-Exon1-Sequenz mit den Intron 1-Sequenzen des Saccharosesynthase-Gens ergeben eine mindestens 1000-fache Erhöhung der Genexpression.

In diesem Zusammenhang wird auf die nachfolgende Tabelle 2 verwiesen, worin die CAT-Aktivitäten der chimären Plasmide pCM 1106 und pCM 1111 der Fig. 4 in Relation zum chimären Referenzplasmid pRT 101 CAT (Aktivität = 1) verglichen werden.

**Tabelle 2**

| pRT 101 CAT | pCM 1106 | pCM 1111 |
|---|---|---|
| 1 | 7,5-11,9 | 913-1203 |

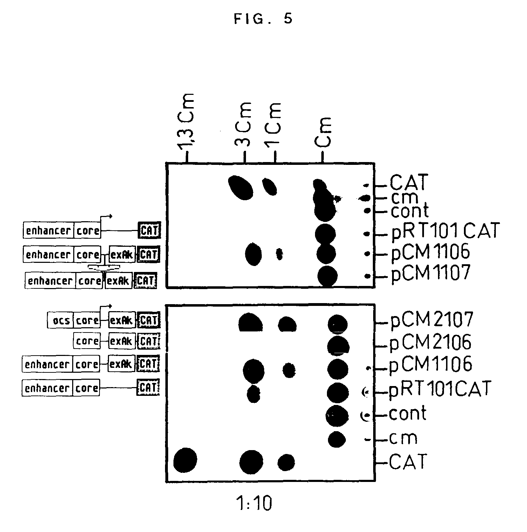

Das Dünnschichtchromatogramm in Fig. 5 zeigt, daß die Akt1-Exon1-Sequenz nicht ohne regulatorische Sequenzen in upstream-Richtung zu einer Erhöhung der Genexpression führt. Die Akt1-Exon1-Sequenz erhöht die Markergenexpression bis auf das mindestens 10-fache, wenn sie in downstream-Richtung des gesamten CaMV 35S-Promotors eingefügt ist (pCM 1106). Dagegen hebt die Deletion des CaMV 35S-Promotor im chimären Plasmid pCM 1106 (pCM 2106, Fig. 1) das Stimulationsvermögen der Akt1-Exon1-Sequenz völlig auf.

Da die Akt1-Exon1-Sequenz die Anwesenheit von regulatorischen Elementen in upstream-Richtung zur effektiven Markergenexpression benötigt, kann die Aktivität des chimären Plasmids pCM 2106 durch Einfügung von regulatorischen Elementen in upstream-Richtung wieder hergestellt werden. So wurde die 18 bp OTF-Bindungsstelle als regulatorische Sequenz in upstream-Richtung des CaMV 35S-Promotors des chimären Plasmids pCM 2106 unter Bildung des chimären Plasmids pCM 2107 eingefügt (siehe auch Fig. 1). Die transiente Genexpression des chimären Plasmids pCM 2107 zeigt, daß die Akt1-Exon1-Sequenz im nicht stimulierenden chimären Plasmid pCM 2106 wieder ihre mindestens 10-fache stimulierende Wirkung entfaltet, wenn das Plasmid die OTF-Bindungsstelle enthält.

Am Beispiel der chimären Plasmide pCM 1106 und pCM 1107 ist zu erkennen, daß die Entfernung zur Transkriptionsstartstelle des heterologen Promotors ebenfalls sehr wichtig ist. Die Deletion von 11 Basenpaaren im Polylinker von pCM 1106, die sich zwischen der Transkriptionsstartstelle und den Akt1-Exon1-Sequenzen befinden, heben die stimulatorische Wirkung des Promotorkonstrukts auf (pCM 1107). Demnach sollte die Entfernung zwischen Transkriptionsstartstelle und Akt1-Exon1-Sequenz bevorzugt mindestens 11 bp betragen.

Die relativen Werte der Erhöhung der Genexpression anhand der in Fig. 5 gezeigten chimären Plasmiden sind aus der nachfolgenden Tabelle 3 zu entnehmen. In dieser Tabelle werden die CAT-Aktivitäten der chimären Plasmide pCM 1106, pCM 1107, pCM 2106 und pCM 2107 in Relation zum chimären Referenzplasmid pRT 101 CAT (Aktivität = 1) in *Hordeum vulgare* Protoplasten verglichen.

**Tabelle 3**

| pRT 101 CAT | pCM 1106 | pCM 1107 | pCM 2106 | pCM 2107 |
|---|---|---|---|---|
| 1 | 8,1-12,4 | 0,8-1,2 | 0,8-1,15 | 8,9-14,5 |

Bei dem erfindungsgemäß eingesetzten modulartigen Promotor-Konstrukt handelt es sich somit um einen ggf. aus einzelnen regulativen DNA-Sequenzen aufgebauten effektiven Promotor, mit dem es möglich ist, die Genexpression fremder Gene in Pflanzenzellen auf das bis zu mindestens 4000-fache zu erhöhen.

Eine zentrale Rolle spielt eine DNA-Sequenz aus dem 5'-nicht translatierten Bereich des Exon 1 des Aktin 1-Gens aus Reis. Diese Sequenz ist ein die RNA Polymerase II stimulierendes cis-Element, das sich in downstream-Richtung der Transkriptionsstartstelle befinden muß und nur in Gegenwart von regulatorischen DNA-Sequenzen in upstream-Richtung seine stimulierende Wirkung auf die Genexpression entfaltet.

## Patentansprüche

1. Pflanze und deren Vermehrungsgut, regeneriert aus einer Pflanzenzelle, die mit einem Vektor transformiert ist, der ein in einer Pflanzenzelle zu exprimierendes Gen und ein modulartiges Promotor-Konstrukt, das einen in Pflanzenzellen wirksamen Promotor und eine DNA-Sequenz aus dem Exon 1 des Aktin 1-Gens aus Reis aufweist, oder die Allele oder die die Genexpression stimulierenden Derivate dieses modulartigen Promotor-Konstrukts enthält mit der Maßgabe, daß der Promotor kein Promotor des Aktin 1-Gens aus Reis ist.

2. Pflanze nach Anspruch 1, dadurch gekennzeichnet, daß die DNA-Sequenz des Promotor-Konstrukts der Position +4 bis +57 im Exon 1 des Aktin 1-Gens entspricht.

3. Pflanze nach Anspruch 1 oder 2, dadurch gekennzeichnet, daß das Promotor-Konstrukt weiterhin eine DNA-Sequenz aus dem Intron 1 des Saccharosesynthase-Gens von Mais enthält.

4. Pflanze nach Anspruch 3, dadurch gekennzeichnet, daß das Promotor-Konstrukt zusätzlich eine 18 bp OTF-Bindungsstelle enthält.
